# EUROPEAN PATENT APPLICATION

(11) **EP 4 311 512 A1**
(43) Date of publication of application: **31.01.2024**
(21) Application number: 22306150.8
(22) Date of filing: 29.07.2022
(51) Int. Cl.: A61B 34/10

(54) **COMPUTER-IMPLEMENTED METHOD OF LIVER RESECTION PLANNING**

(71) Applicant: Guerbet, 93420 Villepinte (FR); Institut National de Recherche en Informatique et en Automatique, 78150 Rocquencourt (FR); Institut National de la Santé et de la Recherche Médicale (INSERM), 75013 Paris (FR); Assistance Publique - Hôpitaux de Paris, 75012 Paris (FR); Université Paris-Saclay, 91190 Gif-sur-Yvette (FR)
(72) Inventor: Ali, Omar, 75013 PARIS (FR); BÔNE, ALEXANDRE, 75009 PARIS (FR); ROHÉ, Marc-Michel, 92120 MONTROUGE (FR); Vibert, Eric, 91190 GIF-SUR-YVETTE (FR); Vignon-Clementel, Irène, 78153 LE CHESNAY CEDEX (FR)
(74) Representative: Regimbeau

(57) **Abstract**

The invention relates to a computer-implemented method of liver resection surgery planning, comprising the steps of:
- processing (E2) preoperative computed tomographic images (10) of a patient to generate liver, liver lesion(ss) and venous vessels segmentations;
- pruning the venous vessels segmentation to retain only major vessels and determining (E3) a primary hepatic zone (PHZ) from the retained major vessels;
- determining (E4) at least one quantitative feature from the primary hepatic zone, the liver segmentation and the liver lesion(s) segmentation;
- processing (E5) the determined quantitative features with a classification model to determine a liver resection complexity score for the patient.

## Description

### TECHNICAL FIELD

The field of the invention is that of surgery planning by preoperative assessment of a surgery operation complexity.

### DESCRIPTION OF RELATED ART

Preoperative prediction of the difficulty of a surgical operation is an important aspect of planning surgery. With the help of accurate prediction, surgeons can schedule time and team for the operation appropriately. Surgeons can also be made aware about the possible complications that may arise in high-risk patients. High-risk patients can be informed accordingly and it may even be decided not to proceed to the operation for such patients.

Liver cancer is a prominent contributor to cancer mortality worldwide, ranking second in the most common cause of cancer-related deaths. In the management of the early stages of liver cancer, liver resection (LR) is the most prevalent type of treatment. With the considerable variations in technicalities of different types of LR, preoperative assessment of resection complexity is necessary to minimize peri- and post-operative complications.

Known scoring systems for LR complexity rely either on qualitative readings or some degree of user interaction to anticipate the LR complexity. While experienced surgeons in specialized medical centres may have the necessary expertise to accurately anticipate LR difficulty and prepare accordingly, an objective method able to reproduce this behaviour would have the potential to improve the routine standard of care and make liver surgery safer.

### BRIEF DESCRIPTION OF THE INVENTION

The invention aims at providing such an objective method and, more particularly, a LR complexity prediction method that would be automated and easily interpretable. In this respect, according to one aspect, the invention relates to a computer-implemented method of liver resection planning, comprising the steps of:
- processing preoperative computed tomographic images of a patient to generate liver, liver lesion(ss) and venous vessels segmentations;
- pruning the venous vessels segmentation to retain only major vessels and determining a primary hepatic zone from the retained major vessels;
- determining at least one quantitative feature from the primary hepatic zone, the liver and lesion(s) segmentations;
- processing the determined at least one quantitative feature with a classification model to determine a liver resection complexity score for the patient.

Certain preferred, but non-limiting aspects of the method are as follows:
- processing the preoperative computed tomographic images to generate the liver, liver lesion(s) and venous vessels segmentations can comprise processing the preoperative computed tomographic images by a first pre-trained neural network to segment the liver and the liver lesion(s) and by a second pre-trained neural network to segment the venous vessels;
- the classification model can be a classifier;
- pruning the venous vessels segmentation comprises:
   ∘ identifying vascular branches and branching networks within the venous vessels segmentation, wherein each branch and branching network has a branch vascular entry;
   ∘ identifying bifurcations within a vascular branching network;
   ∘ pruning said vascular branching network when a pre-set number of vascular bifurcations is reached starting from the branch vascular entry.
- a bifurcation can be identified at a point within a vascular branching network where a branch length or a branch diameter increase above a respective threshold;
- determining the primary hepatic zone can further comprise determining the convex hull based on the vessels retained after pruning;
- the at least one quantitative feature includes position of the liver lesion(s) with respect to the primary hepatic zone;
- the position of the liver lesion(s) with respect to the primary hepatic zone can consist in the occupancy volume of the lesion(s) in the primary hepatic zone when the lesion(s) do intersect the primary hepatic zone, or in an opposite of the minimal distance from the lesion(s) to the primary hepatic zone when the lesion(s) are outside the primary hepatic zone;
- the at least one quantitative feature can further comprise a liver volume, a number of lesion(s) and a volume of lesion(s).

According to other aspects, the invention also relates to:
- a data processing apparatus comprising a processor configured to perform the steps of the method proposed;
- a computer program product comprising instructions which, when the program is executed by a computer, cause the computer to carry out the steps of the method;
- a visualization software platform equipped with said computer program which can be used to visually check the intermediate results, manually amend them or interactively modify hyperparameters, and automatically generate a standardized medical report.

### BRIEF DESCRIPTION OF THE DRAWINGS

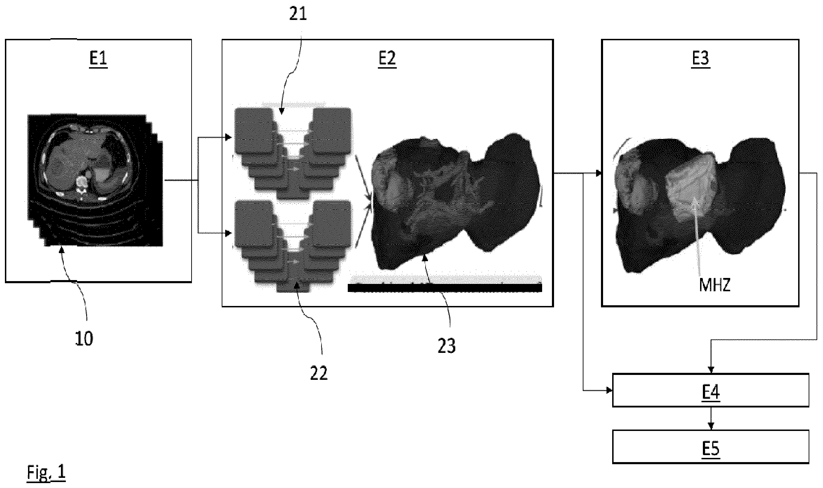

Other aspects, aims, advantages and features of the invention will better appear upon reading the following detailed description of preferred embodiments thereof, provided as a non-limiting example, and done in reference to the appended drawings, in which figure 1 illustrates the various steps of a method in accordance with a possible embodiment of the invention.

### DETAILED DESCRIPTION OF THE INVENTION

The invention relates to a computer-implemented method of liver resection (LR) surgery planning which automatically predicts LR complexity from preoperative computed tomographic (CT) images of a patient.

With reference to figure 1, this method comprises a step E1 of receiving preoperative CT (Computed Tomography) images (also referred as to CT scans) 10 of the patient's liver, typically contrast-enhanced images, which were previously acquired by a CT scanner. Then in a step E2, the received images 10 are processed to generate liver, liver lesion(s) and (portal and hepatic) venous vessels segmentations. In a further step E3, the method comprises pruning the venous vessels segmentation to retain only major vessels and from the retained major vessels, determining a primary hepatic zone (PHZ). The method follows with a step E4 of determining quantitative features from the segmentations and the primary hepatic zone and ends with a step E5 of processing the determined quantitative features with a classification model to determine a liver resection complexity score for the patient.

### Liver, Lesion and Vessel Segmentation

Step E2 in the proposed method is the segmentation of the liver, its lesion(s) and its venous vessels from the portal phase of the preoperative CT images 10 obtained at step E1. This segmentation may provide a binary liver/liver lesion segmentation mask and a binary vessel segmentation mask. Figure 1 shows under reference 23 the combined segmentations of the liver, lesion(s) and vessels.

In a possible embodiment, processing the received images 10 comprises processing the received images by a first pre-trained neural network 21 and by a second pre-trained neural network 22. The first pre-trained neural network 21 is dedicated to segment the liver and liver lesion(s) while the second pre-trained neural network 22 is dedicated to segment the portal and hepatic venous vessels. The first and second pre-trained neural networks 21, 22 may be two independent 3D Convolutional Neural Networks. In a possible embodiment, the first and second pre-trained neural networks 21, 22 each has a U-Net architecture comprising an encoding and a decoding path. More particularly, the first and second pre-trained neural networks 21, 22 may be nnU-Net's as for instance described in F. Isensee et al., "nnU-Net: a self-configuring method for deep learning-based biomedical image segmentation," Nat Methods, 18(2), 203-211 (2021). The first and second neural networks 21, 22 may be trained with the dice and cross-entropy loss function using stochastic gradient descent with Nesterov momentum and a geometrically decaying learning rate.

### Topological Analysis of the Liver Vasculature

It is known that centrally located tumours require more technically challenging surgical resections due to their proximity to the liver's major vessels (portal and hepatic veins), often necessitating vasculature reconstruction which further increases the complexity of the resection. In this respect, the method according to the invention processes the segmented (portal and hepatic) vessels to acquire a detailed knowledge of the morphology and structure of the hepatic vasculature and to derive therefrom the position of the tumours with respect to the major hepatic venous vessels. More particularly, the method comprises at step E3 pruning the venous vessels segmentation to retain only the major vessels and determining a primary hepatic zone (PHZ) from the venous vessels segmentation retained after pruning.

In a possible embodiment, pruning the venous vessels segmentation comprises:
- Identifying vascular branches and branching networks within the venous vessels segmentation, wherein each branch and/or branching network has a vascular entry and wherein a branching network is a tree of branches (i.e. vessels) and comprises a group of branches (i.e. vessels) ;
- identifying bifurcations within the vascular branching networks, where a major bifurcation may be identified at a point within a vascular branching network when a branch length or a branch diameter does not satisfy a respective threshold;
- pruning vascular branches of a branching network when a pre-set number of bifurcations is reached starting from the branching network vascular entry.

After pruning the venous vessels segmentation, the retained vascular branches, comprise only the major vessels, and these are used to define the primary hepatic zone, i.e., a volume circumscribing the major liver vessels. In an embodiment, determining the primary hepatic zone comprises determining the convex hull of the retained vascular branches (i.e., the non-pruned vascular branches).

An example embodiment of step E3 comprises the successive steps listed below, built on the method presented in D. Selle et al., "Analysis of vasculature for liver surgical planning," IEEE Trans. Med. Imaging, 21(11), 1344-1357 (2002).
1. *Skeletonization.* The binary vessel segmentation mask is thinned to reduce each connected component to a skeleton mask, 1-voxel wide, using for instance the method described in T. C. Lee et al., "Building Skeleton Models via 3-D Medial Surface/Axis Thinning Algorithms," CVGIP: Graphical Models and Image Processing, 56(6), 462-478 (1994), available in the Scikit-Image 0.20.0 python package. Local vessel radius values are associated to each skeleton element after the computation of the distance transform of the vessel segmentation mask, using for instance the SciPy 1.8.0 library.
*2. Graph construction.* The skeleton is converted into a graph representation G *=* (*V, E*) with vertices V = {*v*₁, ..., *vₙ*} and undirected edges E = {(*v,* w)| *v, w* ∈ V, *v* ≠ *w*}, considering two voxels as neighbours if their corresponding indexes differ by a maximum value of 1 in each direction. Edge lengths are computed accordingly.
*3. Branch decomposition.* The graph G is decomposed into a series of edge-disjoint subgraphs *B*₁, ..., *Bₘ* that are called branches, such that each edge (*v, w*) ∈ E belongs to one branch exactly. The length *len(B)* and mean radius *rad(B)* of each branch *B* is computed, by summation or averaging of edge lengths and radius respectively.
*4. Vascular entry identification.* Anatomically, the considered liver vasculature is composed of two distinct vessel trees, the portal and hepatic trees. To identify the corresponding liver entry points, the vessel segmentation mask is successively eroded, and the mass centres of the two most persistent connected components are projected onto the skeleton. The closest vertices *vₚ* and *vₕ* with degree 1 are then identified.
5. *Robust and interpretable tree pruning.* Two tree hierarchies are extracted from G using *vₚ* and *vₕ* as respective seeding vertices, retaining only the major connected branches. The branch lengths and diameters are exploited to robustly identify significant branch bifurcation levels *bif*(*B*)*,* in a recursive tree building approach. The vessels extending beyond the third major vascular bifurcation for instance are considered irrelevant for surgical planning and are pruned.
*6. Morphological reconstruction.* The skeletons retained after pruning are inflated by a dilation factor equal to the local diameter values. Numerical round-up and mask intersection with the original segmentation allow to reconstruct the local vessel geometry.
*7. Primary hepatic zone definition.* The convex hull based on the vessels retained after pruning finally defines the primary hepatic zone, where resection surgeries are assumed to be difficult to perform

A possible algorithm for the robust and interpretable tree pruning procedure is as follows.

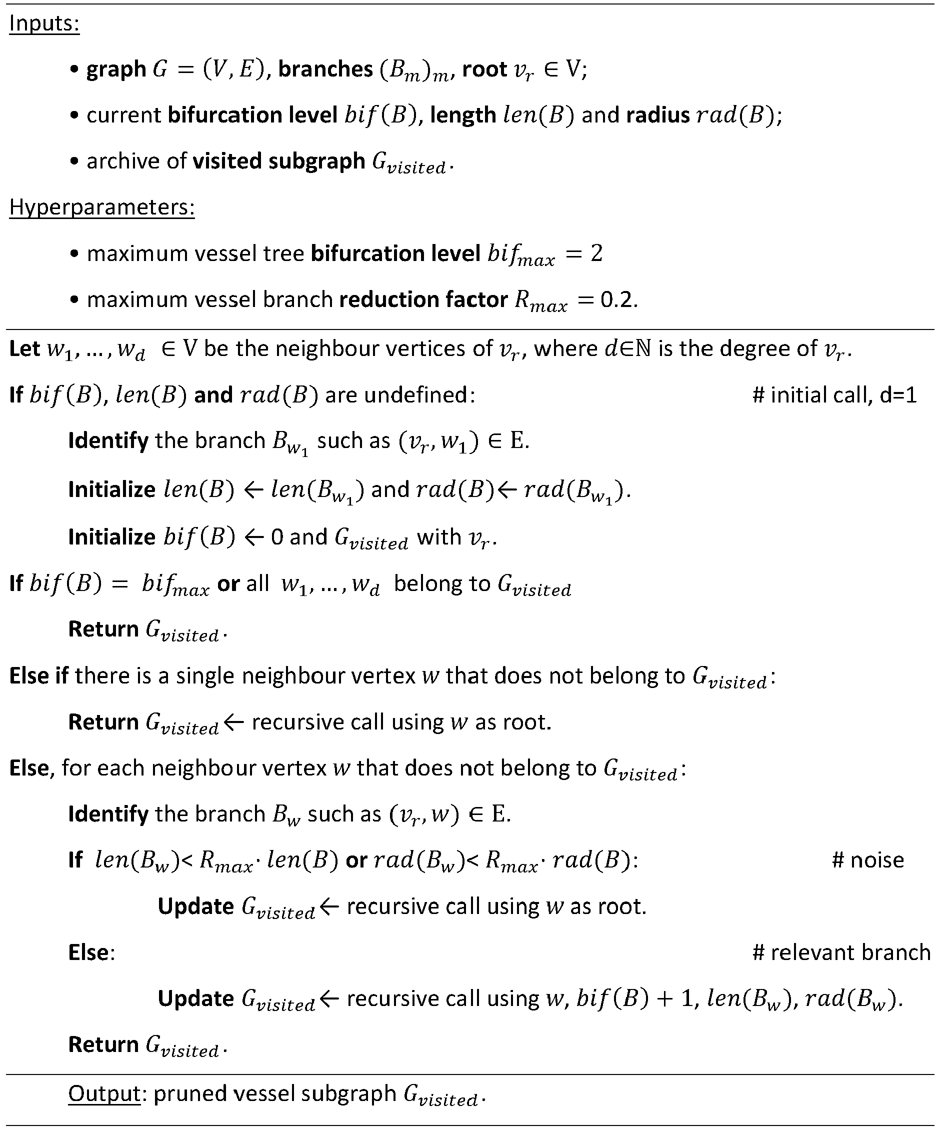

### Quantitative Features for LR Complexity Prediction

As presented above, the method follows with the step E4 of determining quantitative features from the segmentations (including the segmented liver and liver lesion(s)) and the primary hepatic zone and with the step E5 of processing the determined quantitative features with a classification model to determine the liver resection complexity score for the patient. The quantitative features include a first feature F_{PHZ} defined as a relative position of the liver lesion(s) with respect to the primary hepatic zone. For instance, this first feature F_{PHZ} corresponds to the occupancy volume of the lesion(s) in the primary hepatic zone when the lesion(s) do intersect the primary hepatic zone, or corresponds to an opposite of the minimal distance from the lesion(s) to the major vessels in the primary hepatic zone when the lesion(s) are outside the primary hepatic zone.

F_{PHZ} is utilized as a quantitative indicator of the position of the lesion(s) in the liver, which when central and close to the major liver vessels (up to the second generation of bifurcations in the portal tree and the emergence of the three hepatic veins) is known to complexify LRs.

The quantitative features may further include a liver volume V_{Liv}, a number of lesion(s) N_{Les} and a volume of lesion(s) V_{Les}, as determined from the segmented liver and liver lesion(s).

The purpose of using the liver volume VLiv arises from the importance of keeping a viable remnant liver volume post-LR to withstand the bodily functions. Furthermore, the volume V_{Les} and the number N_{Les} of lesion(s) are considered since larger or multiple lesion(s) are generally associated with difficult LR even when favourably positioned in the liver.

Additional quantitative features that may be calculated and used for the LR complexity prediction are as follows.

A first group of features is aimed at characterizing the hepatopathy (i.e., liver function) according to criteria such as:
- volume of the spleen.

This requires segmentation of the spleen, which can be generated either by training a separate neural network, or including it in one of the already trained networks. The use of this parameter is based on medical research that showed a strong correlation between the liver function and the size of the spleen.
- diameter of the portal vein to characterize the portal hypertension based on the vessel segmentations.
- morphological characteristics of the liver (atrophy/hypertrophy of the liver segments, hypertrophy of segment 1).

This require delineating the liver segments. By determining the Couinaud segments, the volume of each segment can be computed allowing to determine the hypertrophy/atrophy of the liver segments and particularly segment 1. The delineation of the Couinaud segments is based on the liver and vessel segmentations.
- liver surface nodularity and liver stiffness to assess the stages of fibrosis.
The liver surface nodularity is based on the gray levels of the CT scans, and the liver stiffness is based on an elastometry measure. A dedicated neural network is used to predict this information.

A second group of features aims at quantifying the distance between the lesions. This information can be extracted from the lesion(s) segmentation, and the distances between all the lesions is computed. The larger the distance between the lesions the more difficult the surgery is, because more liver will have to be removed.

It derives from the following that the quantitative features may be any of the following:
- Liver, lesion, and spleen volumes;
- Distance from the lesions to the primary hepatic zone;
- Distance between the lesions;
- Volume of the different liver segments (Couinaud segments 1 through 8);
- Diameter of the veins;
- Liver stiffness;
- Dilation of the biliary ducts which may require segmentations of the biliary ducts which can be obtained with another new neural network;
- Size of the vascular shunts (which can be segmented together with the vessels);
- Volume of ascites which also requires segmentation with a neural network, and is generally very indicative of a poor liver function.

The classification model may be a binary classifier. It can implement a binary cross-entropy loss function with an L2 regularization on the quantitative morphological features and be trained by means of a logistic regression.

Based on the liver resection complexity score calculated for the patient at step E5, a surgeon may anticipate LR complexity and prepare accordingly. For instance, the surgical staff can be carefully assigned based on the LR complexity and their medical expertise. As a consequence, peri- and post-operative risks can be reduced.

In a possible embodiment, another classification model may be used to derive a type of surgery to be performed or a type of surgery the patient is "eligible" to receive based on at least one qualitative feature, typically based on the location of the lesions with respect to the major vasculature and the distance between several lesions.

### Experiments

IRCAD, LiTS and Medical Decathlon (MD) are public datasets of abdominal CT images with different combinations of liver, lesion, and hepatic vessel reference segmentations. LiTS also presents a validation set (LiTS_{LVS}) with 70 unannotated images for the evaluation of the liver and lesion segmentations. Moreover, an internal dataset of 65 patients (ds_{seg}) with reference vessel annotations is used in the venous vessel segmentation task.

The different datasets are pre-processed using the nnUNet's default pre-processing pipeline which includes a contrast clipping, a z-score normalization and a resampling of the images to their median spacing.

A separate internal dataset (dsᵢₙₜₑᵣₙₐₗ) of 128 patients who underwent LR for hepatocellular carcinoma (the most common type of primary liver cancer) was created between the years 2012-2020, with a LR complexity score attributed after the intervention by the surgeon that performed the LR. The dataset has balanced classes with 63 and 65 LR cases respectively considered as complex and not complex.

The first neural network, dedicated to the segmentation of the liver and liver lesions, is trained on IRCAD and LiTS, and evaluated on LiTS_{LVS}, the benchmark test set for the evaluation of liver and lesion segmentations. The second neural network, dedicated to segmentation of the vessels, is first pre-trained on MD, then fine-tuned on ds_{seg} and IRCAD using a 5-fold cross validation on the hepatic vessels in IRCAD. The performance of both models is evaluated using dice metric.

In inference, the two neural networks are employed on dsᵢₙₜₑᵣₙₐₗ to generate liver, lesion(s), and hepatic venous vessel segmentations. Due to the lacking reference annotations in dsᵢₙₜₑᵣₙₐₗ, the generated segmentations are empirically evaluated by expert liver surgeons. Then, the segmentations are post-processed to extract the quantitative morphological features.

The classification model for LR complexity prediction is trained on dsᵢₙₜₑᵣₙₐₗ using the pre-defined features and the leave-one-out method. An ablation study is carried out to determine the best set of features for the prediction of LR complexity. An iterative backward elimination is performed on the least important feature, starting from the default configuration with the four pre-defined features as inputs. The performance of the models is evaluated using the accuracy, F1, and AUC metrics.

The dice scores obtained on the different benchmark testing sets achieve 96.0%, 70.6% and 79.1% for the liver, lesion(s), and vessel segmentations respectively.

After generating the liver anatomy segmentations, the position of the lesion(s) is assessed with respect to the PHZ using F_{PHZ}.

The results of the ablation study are reported in the below Table.

| F_{PHZ} | N_{LES} | V_{LES} | V_{Liv} | Accuracy | F1 | AUC |
|---|---|---|---|---|---|---|
| ✔ | ✔ | ✔ | ✔ | **0,74** | **0,71** | **0,84** |
| × | ✔ | ✔ | ✔ | 0,70 | 0,68 | 0,78 |
| ✔ | X | ✔ | ✔ | 0,76 | 0,73 | 0,79 |
| ✔ | ✔ | X | ✔ | 0,73 | 0,71 | 0,82 |
| ✔ | ✔ | ✔ | X | **0,77** | **0,75** | **0,84** |
| × | ✔ | ✔ | × | 0,72 | 0,70 | 0,77 |
| ✔ | X | ✔ | × | 0,73 | 0,70 | 0,79 |
| ✔ | v | X | × | **0,78** | **0,78** | **0,81** |
| × | ✔ | × | × | 0,66 | 0,60 | 0,65 |
| ✔ | X | × | × | **0,66** | **0,65** | **0,73** |

The best configuration for classifying the LR complexity combines the first feature F_{HCZ} with the volume of the lesion(s) V_{Les} and the number of lesion(s) N_{Les}. This configuration achieves an accuracy, F1-score, and AUC of 0.77, 0.75, and 0.84 respectively. Additionally, the results show that the feature that can be disregarded for having the least impact on the classification of LR complexity is V_{Liv} followed by V_{Les} and N_{Les}. The results also show that the proposed feature F_{PHZ} is the most critical feature in determining the LR complexity, achieving the best scores in single feature evaluation with an accuracy, F1-score, and AUC of 0.66, 0.65 and 0.73 respectively. The invention is not limited to the above-described method but also extends to a data processing apparatus comprising a processor configured to perform the steps of the above-described method as well as to a computer program product comprising instructions which, when the program is executed by a computer, cause the computer to carry out the steps of the above-described method. The steps of the above-method may be complemented with steps of visually checking the intermediate results (liver, lesion, and vessel segmentation output from the neural networks, and the pruning algorithm output to verify the retained vessels and the primary hepatic zone), manually amending these results or interactively modifying hyperparameters (diameter and length criteria for the vessel pruning algorithm, type (e.g., portal or hepatic) and number of displayed vessel branches, number of displayed lesions), and automatically generating a standardized medical report.

## Claims

1. A computer-implemented method of liver resection planning, comprising the steps of:
- processing (E2) preoperative computed tomographic images (10) of a patient to generate liver, liver lesion(ss) and venous vessels segmentations;
- pruning the venous vessels segmentation to retain only major vessels and determining (E3) a primary hepatic zone (PHZ) from the retained major vessels;
- determining (E4) at least one quantitative feature from the primary hepatic zone, the liver and lesion(s) segmentations;
- processing (E5) the determined at least one quantitative feature with a classification model to determine a liver resection complexity score for the patient.

2. The computer-implemented method of claim 1, wherein processing the preoperative computed tomographic images to generate the liver, liver lesion(s) and venous vessels segmentations comprises processing the preoperative computed tomographic images by a first pre-trained neural network (21) to segment the liver and the liver lesion(s) and by a second pre-trained neural network (22) to segment the venous vessels.

3. The computer-implemented method of one of claims 1 and 2, wherein the classification model is a classifier.

4. The computer-implemented method of one of claims 1 to 3, wherein pruning the venous vessels segmentation comprises:
- identifying vascular branches and branching networks within the venous vessels segmentation, wherein each branch and branching network has a branch vascular entry;
- identifying bifurcations within a vascular branching network;
- pruning said vascular branching network when a pre-set number of vascular bifurcations is reached starting from the branch vascular entry.

5. The computer-implemented method of claim 4, wherein a bifurcation is identified at a point within a vascular branching network where a branch length or a branch diameter increase above a respective threshold.

6. The computer-implemented method of one of claims 1 to 5, wherein determining the primary hepatic zone (PHZ) further comprises determining the convex hull based on the vessels retained after pruning.

7. The computer-implemented method of any one of claims 1 to 6, wherein the at least one quantitative feature includes position of the liver lesion(s) with respect to the primary hepatic zone.

8. The computer-implemented method of claim 7, wherein the position of the liver lesion(s) with respect to the primary hepatic zone consists in the occupancy volume of the lesion(s) in the primary hepatic zone when the lesion(s) do intersect the primary hepatic zone, or in an opposite of the minimal distance from the lesion(s) to the primary hepatic zone when the lesion(s) are outside the primary hepatic zone.

9. The computer-implemented method of one of claims 1 to 8, wherein the at least one quantitative feature further comprises a liver volume, a number of lesion(s) and a volume of lesion(s).

10. A data processing apparatus comprising a processor configured to perform the steps of the method of any one of claims 1 to 9.

11. A computer program product comprising instructions which, when the program is executed by a computer, cause the computer to carry out the steps of the method of any one of claims 1 to 9.

12. A visualization software platform equipped with the computer program of claim 9 which can be used to visually check the intermediate results, manually amend them or interactively modify hyperparameters, and automatically generate a standardized medical report.
